# EUROPEAN PATENT SPECIFICATION

(11) **EP 1 308 521 B1**
(45) Date of publication and mention of the grant of the patent: **08.04.2009**
(21) Application number: 01125914.0
(22) Date of filing: 30.10.2001
(51) Int. Cl.: C12Q 1/68, C12Q 1/70

(54) **A process and a kit for detecting foot-and-mouth disease virus**
Verfahren und Kit zum Nachweis des Maul- und Klauenseucheviruses
Procédé et kit pour détecter le virus de la fièvre aphteuse

(43) Date of publication of application: 07.05.2003
(73) Proprietor: Hong Kong DNA Chips Limited, Kowloon, Hong Kong Special Administrative Region (CN)
(72) Inventor: Yu, Albert Cheung-Hoi, Hong King Special Admistrative Region (CN); Peng, Liang, Hong Kong Special Administrative Region (CN); Ko, Lung-Sang, Hong Kong Special Administrative Region (CN); Lau, Lok-Ting, Hong Kong Special Administrative Region (CN)
(74) Representative: Epping - Hermann - Fischer

(56) References cited:
- WO-A-00/00638
- OLEKSIEWICZ MARTIN B ET AL: "Development of a novel real-time RT-PCR assay for quantitation of foot-and-mouth disease virus in diverse porcine tissues." JOURNAL OF VIROLOGICAL METHODS, vol. 92, no. 1, March 2001 (2001-03), pages 23-35, XP002197112 ISSN: 0166-0934
- HODINKA: "The clinical utility of viral quantitation using molecular methods" CLINICAL AND DIAGNOSTIC VIROLOGY, vol. 10, 1998, pages 25-47, XP002198388
- DATABASE EMBL [Online] EBI; AF026168, 16 August 2000 (2000-08-16) "Foot-and-mouth disease virus O virus strain Chu-Pei complete genome" XP002198389
- TSAI ET AL.: "Molecular epidemiological studies on foot-and-mouth disease type 0 Taiwan viruses from the 1997 epidemic" VET MICROBIOL, vol. 74, no. 3, 2000, pages 207-216, XP002211160 & DATABASE EBI [Online] 27 March 2000 (2000-03-27) TSAI: "FMDV type O isolate Taipei-109 VP1 mRNA" Database accession no. AF095869
- DATABASE EBI [Online] FMDV type O isolate NGR/1/88 VP1 gene, 2 July 2001 (2001-07-02) SANGARE: "Molecular epidemiology of type-O Foot-and-mouth disease virus in West Africa" Database accession no. AF300801 XP002211162
- DATABASE EBI [Online] FMDV type O isolate NGR/3/88 VP1 gene, 2 July 2001 (2001-07-02) SANGARE: "Molecular epidemiology of type-O Foot-and-mouth disease virus in West Africa" Database accession no. AF300803 XP002211163
- DATABASE EBI [Online] FMDV O VP1 gene, 18 December 1998 (1998-12-18) ZHAO: "Nucleotide sequence of VP1 encoding regions of FMDV type O strains isolated from pig " Database accession no. FDI131470 XP002211164
- SAMUEL ET AL.: "Foot-and-mouth disease type O viruses exhibit genetically and geographically distinct evolutionary lineages (topotypes)" JOURNAL OF GENERAL VIROLOGY, March 2001 (2001-03), pages 609-621, XP002211161 & DATABASE EBI [Online] 16 January 2001 (2001-01-16) KNOWLES: "FMDV type O partial mRNA for VP1 gene isolate IRQ/30/2000" Database accession no. FDI303499
- DATABASE EBI [Online] 6 January 1999 (1999-01-06) LAI ET AL.: "Sequence analysis of VP1 gene of FMDV type O outbreak in Taiwan in 1997" Database accession no. AF030259 XP002211165
- DATABASE EBI [Online] FMDV type O isolate NGR/2/88 VP1 gene, 2 July 2001 (2001-07-02) SANGARE: "Molecular epidemiology of type-O FMDV in West Africa" Database accession no. AF300802 XP002211166
- REID ET AL: "PRIMARY DIAGNOSIS OF FOOT-AND-MOUTH DISEASE BY REVERSE TRANSCRIPTION POLYMERASE CHAIN REACTION" J VIROL METHODS, vol. 89, 2000, - 2000 pages 167-176, XP002244066
- COLLINS ET AL: "A METHOD TO DETECT MAJOR SEROTYPES OF FOOT-AND-MOUTH DISEASE VIRUS" BIOCHEM BIOPHYS RES COMM, vol. 297, 2002, - 2002 pages 267-274,

## Description

### Field of the Invention

This invention relates to a kit and process for detecting RNA virus, especially foot-and-mouth disease virus (FMDV).

### Background of the Invention

Foot-and-mouth disease (FMD) is an infectious disease of cloven-footed animal caused by the foot-and-mouth disease virus (FMDV). The disease affects many of the animals that humans depend upon for food and other resources, e.g. cattle, pigs, sheep and goats. Economic losses to the beef, dairy, pork, lamb and wool industries coupled with indirect losses due to damage to the tourism and domestic travel industries imposed by quarantine and travel restrictions would be considerable if an uncontrolled outbreak occurred. Infection with FMDV leads to a severe reduction in an animal's condition. Adult animals generally survive infection but their overall health condition is compromised such that they are no longer economically viable. In addition, the virus may persist in the animal after clinical symptoms have subsided causing them to be a reservoir for repeat infections. Consequently, infected animals must be slaughtered. Furthermore, seropositive animals are currently prohibited from being exported, reducing their value. FMD is often fatal to younger animals especially newborns.

FMDV exists as 7 major subtypes (types O, A, C, Sat-11, SAT-2, SAT-3 and Asia-1) with over 65 identified strains known. The only existing test kit for this disease is produced by the Institute for Animal Health (Pirbright, UK). This institute was completely overwhelmed during the UK outbreak in May 2001 and was unable to provide test kits to other locations around the world until the British infection was under control. The need for alternative detection methods is clear. Other diagnostic test kits for FMDV are currently being developed, e.g. by IAH/Intervet (UK/Netherlands) and United Biomedical, Inc. (NY, USA). However, these test kits share the same potential problem as the kit produced by the Institute of Animal Health - that is, that all these tests detect antibodies to the virus produced as a result of infection or vaccination. Consequently, recently infected but seronegative animals will not be identified. Cross-border transport of FMDV seropositive animals is prohibited under international health guidelines. The severe economic constraints that this imposes on farmers, insurance companies and governments are huge. A detection kit based on the identification of viral nucleic acid instead of antibodies may serve to correctly identify infected and uninfected animals and so prevent widespread and unnecessary slaughter of animals with a potential saving of millions of dollars.

Many methods for viral identification are currently used, including cell culture, fluorescent antibody and enzyme immunoassay, and reverse transcriptase polymerase chain reaction (RT-PCR). However, these methods all share the same problems - they have relatively low sensitivity and low specificity. In addition, the detection time may be too long for routine detection purposes, and such methods are relatively difficult to be utilised. Furthermore, as the target of an immunodiagnostic assay is usually a specific protein, the underlying genetic nature of a target may not be obtained directly. Finally, the initial derivation of antibodies is ultimately dependent upon the antigenicity of the protein in the host animal and therefore, cross-reactivity may occur between similar proteins.

Several different FMD vaccines are available although veterinary experts are divided over the utility of vaccination in preventing the spread of FMD. Many authorities recommend the use of FMD vaccine and combined with a rapid, sensitive and accurate detection method for FMDV they may provide a useful alternative to preventing and containing an outbreak of FMD than mass slaughter.

Specific reports related to this application include US5783391 (Rossi et al), which describes a NASBA-based detection system. However, US5783391 is directed at HIV not FMDV. Similarly, many protocols and publications have documented various methods for detecting FMDV (e.g. Beck & Strohmaier, J Virol 1987;61:1621-1629; Samuel et al, Epidemiol Infect 1988;101:577-590; Rodriguez et al, Virology 1992; 189:363-367; Armstrong et al, Vet Microbiol 1994;39:285-298; Callens & De Clercq, J Virol Methods 1997; Pattnaik et al, Acta Virol 1997;41:333-336; Reid et al, J Virol Methods 2000;89:167-176 and Oleksiewicz et al, J Virol Methods 2001;92:23-35; 67:35-44), including RT-PCR and serological methods. However, none of these documents teach the method of the present invention nor use the methodology of US5783391 against FMDV.

A publication in the "Journal of Virological Methods" 92 (2001), pages 23 to 35 discloses a realt-time RT-PCR assay for quantitation of food-and-mouth disease virus. A publication in the journal "Clinical and Diagnostic Virology" 10 (1998), pages 25 to 47 discloses the clinical utility of viral quantitation using molecular methods inter alia nucleic acid sequence based amplification NASBA.

### Object of the invention

Therefore, it is an object of this invention to provide a process and a kit therefor for detecting FMDV, such that the sensitivity and specificity may be improved.

Another object of this invention is to provide a process and a kit therefor for detecting FMDV, such that the detection time and the overall costs for detection may be reduced.

It is yet another object of this invention to provide the isolated and purified primer nucleic acid sequences useful in the process of the present invention.

### Summary of the invention

In one aspect, the present invention provides a process for detecting FMDV in a biological sample, which comprises the following steps
(A) isolating the RNA molecules of the FMDV from the biological sample;
(B) subjecting the RNA molecules of the FMDV to amplification based on a NASBA method to obtain an amplified product containing a nucleic acid sequence complementary to at least a portion of the RNA sequence of FMDV, wherein the NASBA-based amplification of the RNA of the FMDV comprises:
   (a) annealing primer A to the isolated RNA molecules resulted in step (A), wherein primer A includes:
      (i) a first DNA sequence for binding to at least a portion of the RNA sequence of FMDV,
      (ii) a second DNA sequence encoding a promoter DNA sequence of an RNA polymerase;
   (b) allowing the primer A to extend under the presence of a reverse transcriptase to obtain an RNA-DNA hybrid;
   (c) digesting the RNA sequence in the RNA-DNA hybrid obtained in step (b) with an RNase to obtain a single strand DNA molecule;
   (d) annealing the primer B to the single strand DNA obtained in step (c), wherein primer B includes:
      (i') a first DNA sequence for binding to at least a portion of the RNA sequence of FMDV,
      (ii')optionally, a second DNA sequence encoding a detectable DNA sequence ;
   (e) allowing the primer B to extend under the presence of a reverse transcriptase to obtain a double strand DNA molecule;
   (f) subjecting the double-strand DNA obtained in step (e) to amplification under the presence of an RNA polymerase to obtain an amplified RNA product;
(C) detecting the amplified RNA products;
(D)
   wherein the primer A comprises either one of the DNA sequence as set forth in SEQ ID Nos. 1, 2, 3, 4, 5 and 6, and wherein the primer B comprises either one of the DNA sequence as set forth in SEQ ID Nos. 8, 9, 10, 11, 12 or 13.

In another aspect, the invention also provides a kit for detecting FMDV in a biological sample, which comprises:
(A) an isolating agent for isolating the RNA molecules of FMDV from the biological sample;
(B)an amplifying agent for amplifying the isolated RNA molecules to obtain an amplified RNA product, wherein the amplified molecule includes a nucleic acid sequence complementary to at least a portion of the RNA sequence of FMDV, wherein the amplifying agent includes:
   (a) primer A which includes:
      (i) a first DNA sequence for binding to at least a portion of the RNA sequence of FMDV, wherein the first DNA sequence of primer A comprises a DNA sequence as set forth in SEQ ID Nos. 1,2 , 3, 4, 5 or 6; and/or
      (ii) a second DNA sequence encoding a promoter DNA sequence of a RNA polymerase;
   (b) primer B which includes:
      (i) a first DNA sequence encoding at least a portion of the RNA sequence of FMDV, wherein the first DNA sequence of primer B comprises a DNA sequence as set forth in SEQ ID Nos. 8, 9, 10, 11, 12 or 13; and
      (ii) optionally, a second DNA sequence encoding a DNA sequence for binding to the detection molecule, wherein the second DNA sequence of primer B comprises a DNA sequence as set forth in SEQ ID No. 14; and/or
   (c) a reverse transcriptase; and/or
   (d) an RNase; and/or
   (e) a RNA polymerase;;
(C) a detecting agent for detecting the target RNA molecule.
   In a further aspect, the present invention also provides the isolated and purified forms of the primers used in the invention and the sequences complementary thereof.

### Brief description of the drawings

A preferred embodiment of this invention will now be described with reference to the following figures:
Figure 1 shows the flow chart of the overall procedures of the detection of FMDV by the process of this invention.
Figure 2 shows the detailed procedures for the detection of FMDV by the detection process of this invention.

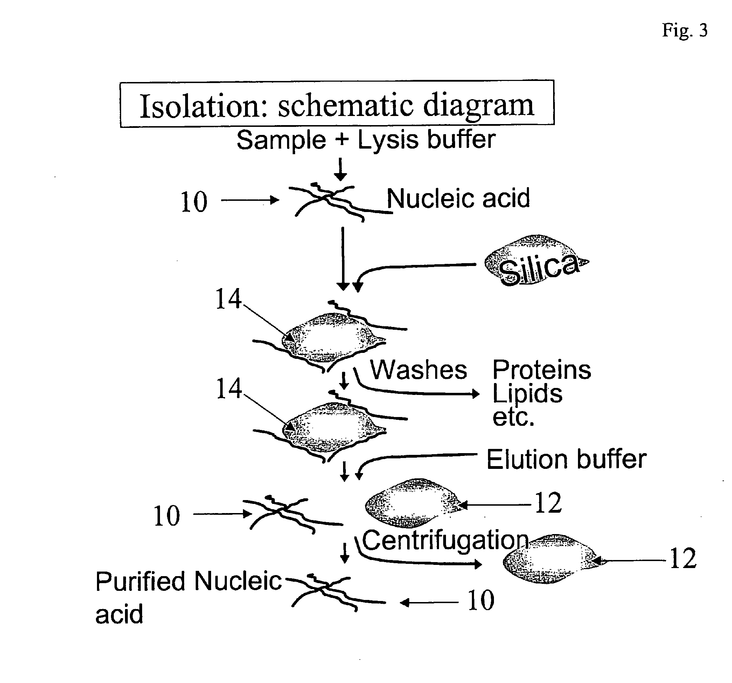

Figure 3 shows the isolation of the viral RNA molecules from the biological sample.
Figure 4 shows the amplification of a portion of the FMDV RNA molecule by two DNA molecules, primers A and B.
Figure 5 shows the immobilisation of the amplified RNA molecule while bound to a detection probe.

### Detailed description of preferred embodiments

Preferred embodiments of this invention are now described with reference to the figures. List 1 is a part list so that the reference numerals in the figures may be referred to easily.

| **Reference No.** | **Description** |
|---|---|
| 10 | nucleic acids in sample |
| 12 | silica |
| 14 | nucleic acids/silica mixture |
| 20 | FMDVRNA |
| 22 | Primer A |
| 24 | Extended Primer A |
| 26 | Extended Primer A(DNA):FMD viral RNA hybrid |
| 28 | Primer B |
| 30 | double-stranded DNA copy of FMDV |
| 32 | target RNA molecule |
| 40 | detection probe |
| 41 | signal generator |
| 42 | capture probe |
| 44 | immobiliser |

The present invention provides a process for detecting FMDV in a biological sample, which comprises the following steps:
(A) isolating the RNA molecules of the FMDV from the biological sample;
(B) subjecting the RNA molecules of the FMDV to amplification to obtain an amplified RNA molecule containing a nucleic acid sequence complementary to at least a portion of the RNA sequence of FMDV;
(C) detecting the amplified RNA products.

The RNA molecules of the FMDV can be isolated by any of the well-known methods in the art. Preferably, a silica method can be used to isolate the RNA of FMDV from the biological sample.

The concentration of FMDV in a biological sample, for example bovine blood, may be very low such that detection of the presence of FMDV RNA may not be performed on the biological sample directly. In order to increase the number of the viral RNA molecules to a sufficient amount for the detection purpose, a suitable amplification technology is required. Nucleic acid sequence-based amplification (NASBA) is known to be a flexible technology with particular use for the amplification of RNA. The amplified RNA molecules may then be detected by suitable technology. NASBA is a rapid, highly sensitive and highly specific method for the detection of FMDV. Results can be obtained in as little as one day. In addition, it can discriminate between different subtypes of the FMDV strains directly.

FMDV contains its genetic material in the form of a single strand of viral ribonucleic acid (RNA). FMDV RNA contains the genes necessary for its reproduction and one of the essential genes is called VP1. This gene is approximately 626 to 638 nucleotides in length depending on the strain studied, with the nucleotides numbered from the 5' end of the molecule. Another important region of the FMDV genome is called IRES (internal ribosome entry site). This region is approximately 450 nucleotides in length, with the nucleotides numbered from the 5' end of the molecule. These essential genes are suitable targets for various detection strategies. Other viral genes can also be targeted.

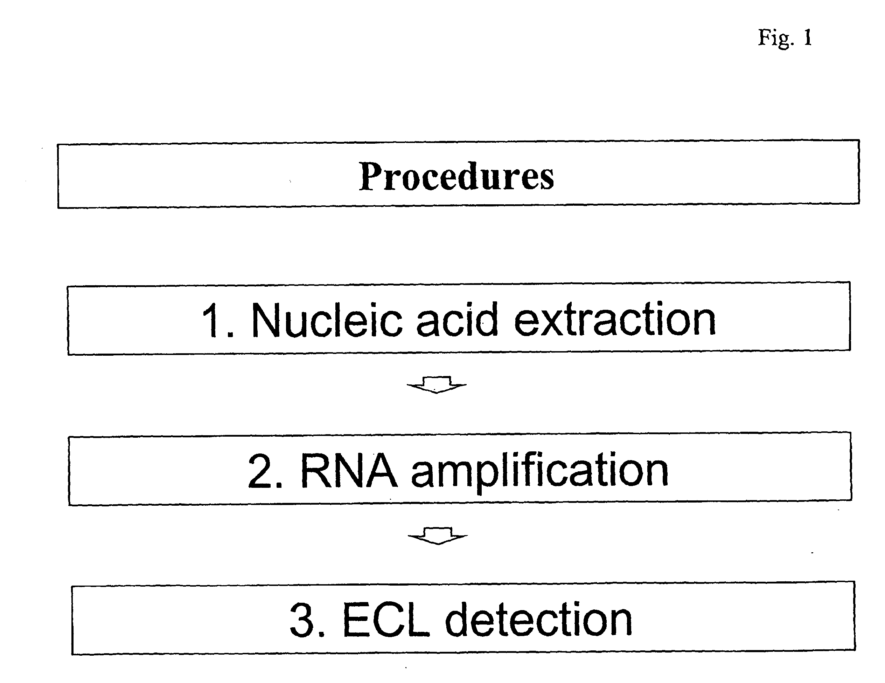

Figure 1 shows the overall procedures for the detection of FMDV by the process of the invention. As shown in Figure 1, the target FMDV nucleic acid molecule, which is in the form of a single strand of RNA, is firstly extracted from a biological sample. The compatible biological sample types may include blood, serum/plasma, peripheral blood mononuclear cells/peripheral blood lymphocytes (PBMC/PBL), sputum, urine, faeces, throat swabs, dermal lesion swabs, cerebrospinal fluids, cervical smears, pus samples, food matrices, and tissues from various parts of the body including brain, spleen, and liver. Other samples that have not been listed may also be applicable.

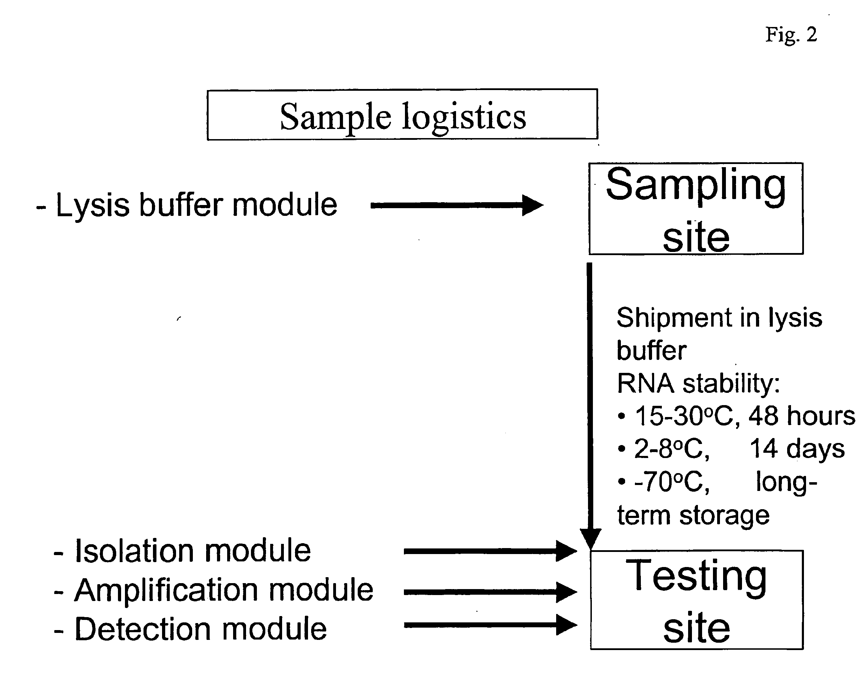

The FMDV RNA molecules may be isolated from the biological sample by applying an isolating agent to the biological sample. Suitable isolating agents may be an adsorbent, for example, silica. Preferably, a lysis agent may be applied before the isolating agent. The lysis agent, for example, a lysis buffer, is responsible for dissolving the proteins and lipids, and denaturing the proteins in the biological sample such that these materials may be removed from the sample more easily. Furthermore, the lysis agent may also serve as a buffer for stabilising the RNA molecule for long-term storage purposes. As shown in Figure 2, the RNA molecule may be stable in the lysis buffer for up to 48 hours at room temperature and may be stored indefinitely at -70°C. The advantages for doing so are that it may not be necessary to perform the analysis at the sampling site, which may not be suitable for carrying out such processes.

An example of suitable lysis buffer may include 5M guanidine thiocyanate and Tris/HCl. The lysis buffer is not part of the invention and the compositions suitable for its purpose are well known in the art. Therefore, the detailed composition of the lysis buffer will not be discussed here. Lysis agents having different compositions that can still achieve the purposes of dissolving proteins and lipids, denaturing proteins, and stabilising the RNA molecules may be utilised in the process of this invention.

After the lysis agent has been applied to the biological sample, the next step is the isolation of the nucleic acid molecules from the sample through the use of an isolating agent. After the lysis agent is applied to the biological sample, nucleic acids (10) together with other unwanted components are in the form of a solution. As the isolating agent, an adsorbent, for example silica (12), may then be added into the solution to adsorb the nucleic acids (10), resulting in a nucleic acids/silica mixture (14). After that, proteins and lipid and other unwanted materials in the solution may be washed away by suitable eluents, for example, 5M guanidine thiocyanate and Tris/HCl solution, Tris/HCl solution, 70% ethanol, or acetone, or their combinations. After removal of proteins, lipids and other unwanted materials the mixture (14) is washed with sufficient amount of eluents, and the nucleic acids (10) released from the silica (12) may then be isolated by centrifugation.

After the target FMDV RNA molecule is extracted from the biological sample, the amount of RNA molecules in the sample may not be sufficient to be detected. Therefore, a portion of the FMDV RNA molecule is subject to amplification to obtain the amplified RNA product by an appropriate amplification technique, for example, NASBA. The amplified RNA product may then be detected by suitable methods.

After the nucleic acids (12) contained in the sample are isolated, they may then be subjected to amplification, for example using the NASBA technique. Two purified and isolated DNA molecules are designed for the amplification purpose, which are termed primers A and B.

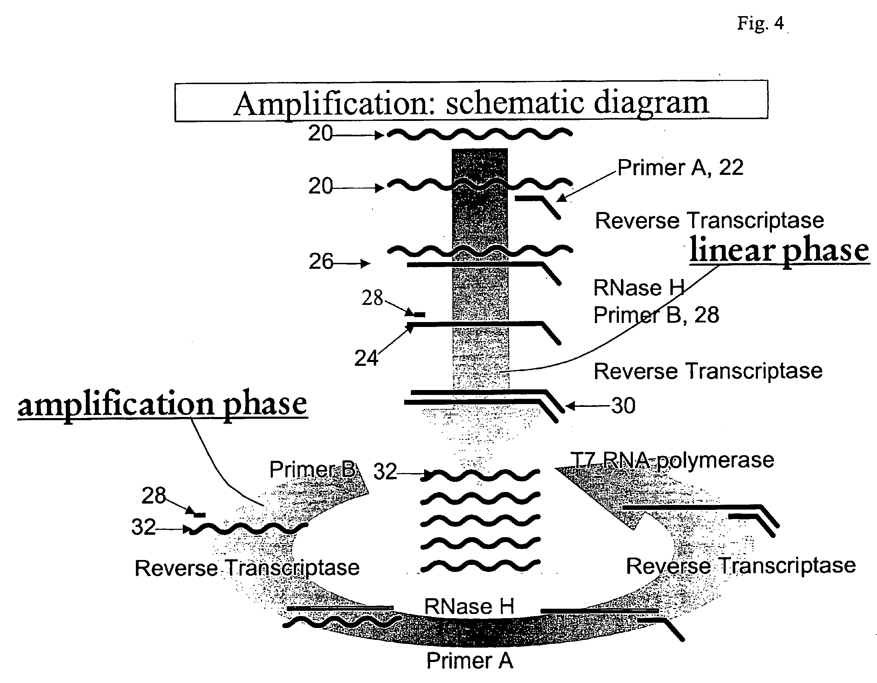

Figure 4 shows a schematic diagram for the amplification of the FMDV RNA by NASBA in this invention. As shown in the figure, the amplification process is initiated by the annealing of primer A (22) to the target FMDV RNA (20), which is a single-stranded RNA molecule. The primer A (22) is designed such that it is capable of binding to the targeted RNA molecule, and further includes a DNA sequence encoding the promoter for a RNA polymerase, preferably bacteriophage T7 RNA polymerase. The precise location of binding depends upon the strain of virus examined. The binding site may change after a certain period of time. The important technical feature of primer A is that it remains capable of binding to a portion of FMDV nucleic acid.

Accordingly, primer A (22) includes a binding sequence encoding a DNA sequence complementary to at least a portion of the FMDV RNA (20). For the purpose of this invention, it is found that the region suitable for binding in the FMDV RNA (20) is a region between nucleotides 551 to 570 of the IRES region of FMDV (type organism for this study is FMDV Type O, GenBank Accession number NC_002527.1), which is found to contain the least number of nucleotides for the binding function. Therefore, the binding sequence of primer A preferably includes a DNA sequence that is complementary to region between nucleotide 551 to 570 of the IRES region of FMDV, which is set forth in SEQ ID No. 1. It should be noted that SEQ ID No. 1 is formally written in the 5'-3' direction. As a result, the orientation of binding with respect to the viral gene is from "back" to "front". This sequence also includes a six nucleotide purine-rich extension at the 5'-end of the molecule that serves to enhance the binding of the primer to the amplicon following the first round of amplification.

As an alternative, nucleotides 501 to 620 (SEQ ID No.2) or nucleotides 481 to 640 (SEQ ID No.3) of the IRES region of FMDV may be used for the binding purpose in primer A (22).

As an alternative, nucleotides 931 to 949 (SEQ ID No.4), nucleotides 881 to 999 (SEQ ID No.5), or nucleotides 861 to 1019 (SEQ ID No.6) of the IRES region of FMDV may be used for the binding purpose in primer A (22).

For the amplification purpose primer A (22) further includes a DNA sequence encoding a promoter of a RNA polymerase, for example bacteriophage T7 RNA polymerase. A suitable promoter DNA sequence is set forth in SEQ ID No.7 when T7 RNA polymerase is used. The promoter sequence is preferably attached to the 5' end of the binding sequence, such that the binding sequence may extend at the 3' end when primer A binds to FMDV RNA. If other RNA polymerases are utilised, the promoter sequence will have to be changed accordingly.

After primer A binds to the FMDV RNA, primer A is extended through the action of a suitable reverse transcriptase, for example Avian Myeloblastosis Virus-Reverse Transcriptase (AMV-RT) in the presence of suitable nucleotides at the 3' end of primer A. Therefore, an extended primer A (24) including the following sequences is resulted:
(a) a DNA sequence that is complementary to a portion of FMDV RNA; and
(b) a DNA sequence encoding a promoter for a RNA polymerase.

The FMDV RNA portion of the resulting DNA:RNA hybrid (26) is eliminated through the action of an RNase, preferably, the enzyme Ribonuclease-H (RNase-H). This allows for the primer B (28) to anneal to the extended primer A (24) at a position that is upstream from the primer A (22) annealing site. Therefore, in order for the primer B (28) to bind to the extended primer A (24), primer B (28) includes a first binding DNA sequence encoding a portion of the FMDV IRES region sequence. Preferably, this first DNA sequence of primer B (28) encodes nucleotides 341 to 359 of the IRES region of FMDV (SEQ ID No.8). The type organism for these studies is FMDV Type O, GenBank Accession number NC_002527.1. As an alternative, nucleotides 291 to 409 (SEQ ID No.9) or nucleotides 271 to 429 (SEQ ID No. 10) of FMDV VP1 gene may be utilised.

As an alternative, nucleotides 251 to 269 (SEQ ID No.11), nucleotides 201 to 319 (SEQ ID No.12), or nucleotides 181 to 339 (SEQ ID No.13) of FMDV IRES region may be utilised.

To achieve the detection purpose, primer B (28) may further include a second DNA sequence that is complementary to the nucleic acid sequence of a detection molecule. If the detection molecule is designed in a way such that it includes a DNA sequence encoding a portion of the RNA sequence of FMDV such that it may bind to the amplified RNA products, it may not be necessary for primer B to include the second DNA sequence. In this case, primer B may consist of merely the first DNA sequence.

Preferably, a second DNA sequence encoding the DNA sequence set forth in SEQ ID No.14 is included in primer B. The second DNA sequence is preferably attached to the 5' end of the binding sequence of primer B. SEQ ID No. 14 is subject to change if other detection nucleic acid sequences are used.

After the annealing of primer B (28) to the extended primer A (24), primer B (28) extends down through the RNA polymerase promoter at the end of the extended primer A (24) through the action of reverse transcriptase. As a result, a double-stranded DNA copy (30) of the original FMDV RNA target sequence is produced, encoding an intact RNA polymerase promoter at one end and a portion of FMDV RNA sequence at the other end. This promoter is then recognised by an RNA polymerase, resulting in the production of large amounts of amplified products (32) that includes an RNA sequence complementary to a portion of the original FMDV RNA sequence.

The product of the amplification process is a large quantity of amplified products (32) each containing the following RNA sequences:
(a) a RNA sequence complementary to a portion of the original FMDV RNA; and
(b) a RNA sequence complementary to the nucleic acid sequence of a detection molecule, if primer B includes the corresponding second DNA sequence.

The amplified product (32) of this particular embodiment further includes a RNA sequence encoding the promoter for T7 RNA polymerase, which is automatically included during the amplification process by the T7 RNA polymerase. However, this segment of RNA sequence may have no function in the detection step.

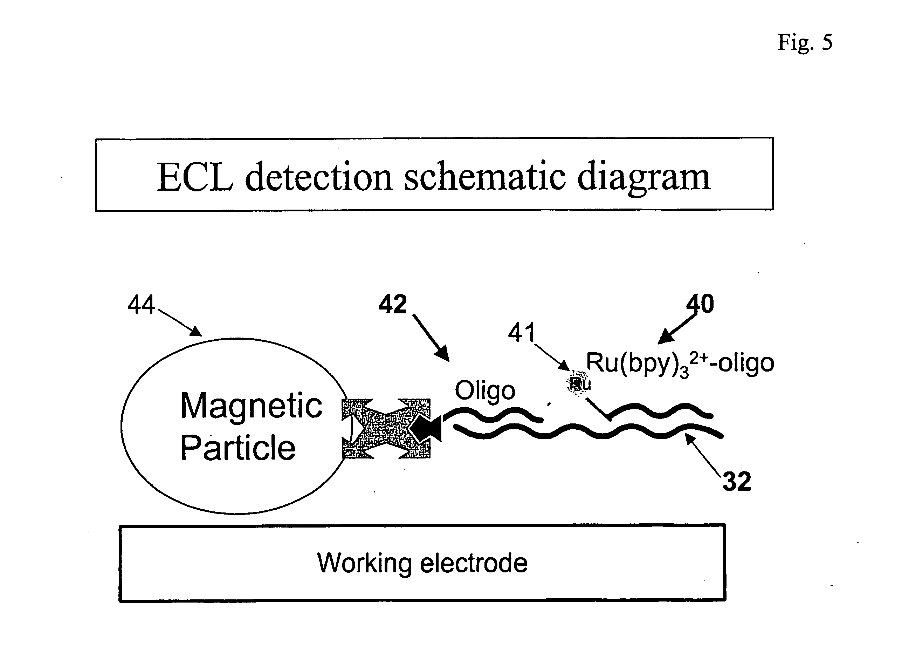

The detection of the amplified target RNA molecule (32) is illustrated in Figure 5. The amplified RNA molecule (32) may be detected by binding to the detection molecule that is capable of generating a signal, for example a detection probe (40). The signal may be generated from a signal generator (41) that is attached to the detection probe (40). In a particular preferred embodiment as shown in Figure 5, the signal generator (41) is a ruthenium-bipyridine complex [Ru(bpy)₃]²⁺. As an alternative, the signal generator (41) may be radioactive (e.g. ³²P), chemiluminescent (e.g. luciferin/luciferase), fluorescent (e.g. fluorescein), enzymatic (e.g. alkaline phosphatase, horseradish peroxidase, beta-glucuronidase), or other electrochemiluminescent molecules.

If primer B includes the corresponding second DNA sequence that is complementary to a detection DNA sequence, the amplified RNA molecule (32) includes a RNA sequence complementary to the nucleic acid sequence of the detection molecule. The advantage of utilising such a design is that commercially available detection molecules may be used.

If primer B consists of merely the first DNA sequence that encodes a portion of the FMDV RNA, a new detection molecule is required. In this case, the detection molecule may include:
■ a nucleic acid sequence encoding a portion of FMDV RNA sequence that is complementary to that encoded in the amplified RNA molecule (32); and
■ a signal generator.

The amplified RNA molecule (32) is contained in a mixture together with other undesired components including the unamplified nucleic acids contained in the original sample, the primers A and B, the unreacted nucleotides, and most importantly, the unbound detection molecules. Therefore, the target RNA molecule (32) may be immobilised by a capture molecule, for example the capture probe (42), such that the undesired components may be washed away. The capture probe (42) is capable of binding to the amplified RNA molecule (32). This may be achieved by including a nucleic acid sequence encoding a portion of the FMDV RNA sequence that is complementary to that encoded in the amplified RNA molecule (32). The capture probe (42) is further attached to an immobiliser (44), which may immobilise the RNA molecule (32) so that other undesired components may be washed away. The immobiliser (44) as shown in Figure 5 is a magnetic particle that may be attracted to a working electrode. Other immobilisers may also be utilised, for example, a piece of polymer to which a number of capture probes (42) are attached.

When using primers A and B to detect all subtypes of FMDV, preferably, the first DNA sequence of the capture probe encodes nucleotides 520 to 544 of the IRES region of FMDV (SEQ ID No.24, Figure 29). The type organism for these studies is FMDV Type O, GenBank Accession number NC_002527.1. As an alternative, the capture probe can encompass any region of the target RNA molecule defined by the ends of the primers A and B, excluding that portion where the detection molecule binds.

The sequence of the detection probe may be complementary to any region of the amplified RNA product whose ends are defined by primers A and B . However, the detection probe sequence cannot overlap that of the capture probe, as this would affect the interaction of the amplified RNA with the capture probe and vice versa.

As shown in Figure 5, the amplified RNA molecule may be immobilised together with the detection probe (40). Therefore, there may be no restriction on the timing of the addition of the capture probe (42) into the mixture. The capture probe (42) may be added after or before the addition of the detection probe (40), as long as the capture probe is added before the washing step.

Given the nucleic acid sequences of primers A and B, and the capture probe, the synthesis of the corresponding complementary DNA molecules will be apparent to one skilled in the art. Such complementary DNA molecules may be used as templates in the synthesis of primers A and B , and the capture probe.

The present invention is now illustrated by the following non-limiting examples. It should be noted that various changes and modifications could be applied to the following example and processes without departing from the scope of this invention. Therefore, it should be noted that the following example should be interpreted as illustrative only and not limiting in any sense.

### Example

### Material and Methods

### 1. Virus strains and cell culture

FMDV isolates were obtained by preparing 10% suspensions (W/V) of buffalo (Syncerus caffer), impala (Aepyceros melampus) and bovine probang and epithelial specimens according to standard procedures. Primary pig kidney cell (PK) were inoculated with these suspensions and propagated further on IBRS2 (Instituto Biologico Rim Suino) or BHK (baby hamster kidney) cells.

### 2. Synthesis of primers:

The primers are designed by the named inventors and synthesized by Bioasia Co. (Shanghai, China).

The detailed components of the detection kit of this example as follows.
**A. Lysis buffer and preparation thereof**
   ■ 50 x 0.9 ml Lysis buffer (5M guanidine thiocyanate, Triton X-100 Tris/HCl)
**B. Nucleic acid isolation components and preparation thereof**
   ■ 5 x 22 ml Wash Buffer (5M guanidine thiocyanate, Tris/HCl)
   ■ 5 x 0.8 ml Silica (Hydrochloric acid-activated silicon dioxide particles)
   ■ 5 x 1.5 ml Elution buffer (Tris/HCl)
**C. Nucleic acid amplification components and preparaion thereof**
   ■ 5 x 60 µl Enzyme solution (Avian Myeloblastosis Virus-Reverse Transcriptase (AMV-RT), Ribonuclease-H (RNase-H), T7 RNA polymerase stabilised with bovine serum albumin)
   ■ 5 x 10 mg Reagent spheres (lyophilised spheres with nucleotides, dithiothreitol and MgCl₂). Contained in a foil pack with silica gel desiccant
   ■ 1 x 0.6 ml Reagent sphere diluent (Tris-HCl, 45% DMSO)
   ■ 1 x 1.6 ml KCl solution
   ■ 1 x 70 µl FMDV-primer mixture
**D. Nucleic acid detection components and preparation thereof**
   ■ 1 x 0.9ml Generic ECL detection probe (Ruthenium-labelled DNA oligonucleotide Preservative: 5 g/L 2-chloroacetamide)
   ■ 1 x 0.7 ml FMDV-capture probe (Biotinylated-oligonucleotide Preservative: 5 g/L 2-chloroacetamide)
   ■ 2 x 1.7 ml Instrument Reference Solution (Ruthenium-labelled paramagnetic beads)
**E. Positive control and preparation thereof**
   Υ 1 x 1 ml FMDV type O total RNA.

The materials listed above are intended to be used for 50 test reactions.

The replication and the detection of the RNA molecules of the FMDV are described in detail as follows.

### RNA amplification in vitro

**A. Nucleic acid release and isolation**
   1. The Lysis buffer tubes were pre-warmed for 30 min and vortexed regularly before starting nucleic acid release.
   2. The Lysis buffer tubes were centrifuged for 30 sec at 10,000 x g.
   3. 100 µl target RNA was added to the Lysis buffer tubes and was vortexed. The positive control tube was prepared by adding 0.1 ml FMDV total RNA solution to a separate tube. The negative control tube was prepared by adding 0.1 ml distilled water to a separate tube.
   4. The Silica suspension was vortexed and 50 µl of the Silica suspension was added to each sample RNA/ Lysis buffer tube.
   5. The RNA/Lysis buffer/Silica tubes was incubated for 10 min at room temperature (vortexed tubes every 2 min to prevent silica from settling to the bottom).
   6. The RNA/Lysis buffer/Silica tubes were centrifuged for 30 sec at 10,000 x g.
   7. The supernatant (does not disturb the pellets) was carefully removed and 1 ml Wash buffer was added to each tube.
   8. The tubes were vortexed until the pellets had been resuspended completely.
   9. The tubes were centrifuged for 30 sec at 10,000 x g.
   10. Repeated steps (7) to (9)
      ■ Once with Wash buffer
      ■ Twice with 70% ethanol
      ■ Once with acetone.
   11. After the final wash step, any residual acetone was carefully removed with a 100 µl pipette.
   12. The silica pellets was dried in open test tubes for 10 min at 56°C on a heating block.
   13. When dry, 50 µl Elution buffer was added to each test tube.
   14. The tubes were vortexed until the pellets had been resuspended completely.
   15. The resuspended silica was incubated for 10 min at 56°C to elute the nucleic acid (the test tubes was vortexed after 5 min).
   16. The tubes were centrifuged for 2 min at 10,000 x g.
   17. 5 µl of each of the nucleic acid supernatants was transferred to a fresh tube.
**B. Nucleic acid amplification**
   1. For each FMDV RNA reaction, 5 µl of the nucleic acid extract was pipetted into a fresh test tube.
   2. 10 µl of the FMDV RNA-specific amplification solution was added into the test tube. The amplification solution contained primers A and B (SEQ ID Nos. 1 and 8) for the detection of FMDV.
   3. The tubes were incubated for 5 min at 65°C in a heating block.
   4. The tubes were cooled for 5 min at 41°C in heating block.
   5. 5 µl of Enzyme solution was added and was mixed well by flicking the test tube with finger.
   6. The test tubes were immediately returned to 41°C for 10 min.
   7. The tubes were briefly centrifuged and incubated for 90 min at 41°C in a water bath.
**C. Nucleic acid detection**
   1. The detection probe was vortexed until opaque. 20 µl of the amplified RNA product was added to each of the tubes.
   2. For the amplification reactions:
      ■ 5 µl FMDV RNA amplification reaction product was added to the tubes.
      ■ The hybridisation tubes were covered with adhesive tape.
      ■ The hybridisation tubes were mixed until an opaque solution formed.
   3. The adhesive tape was used to cover the hybridisation tubes. This was to prevent evaporation and contamination.
   4. The hybridisation tubes were incubated for 30 min at 41°C.
   5. 300 µl assay buffer was added to each hybridisation tube.

   The resulted samples were detected on a Electro Chemiluminescent Reader (NucliSens ECL Reader, Organon Teknika Inc, Boxtel, Netherlands).

The results of FMDV detection are listed in the following tables. The results are confirmed by DNA sequencing using a Perkin Elmer ABI 310 Genetic Analyser.

### RESULTS

**Table 1, detection of various FMDV subtypes using the universal primer pair (SEQ ID No. 1 plus SEQ ID No. 8).**

| **Sample ID** | **Result** |
|---|---|
| Reference Standard | |
| Assay negative | |
| SAT-1 | Positive |
| SAT-2 | Positive |
| SAT-3 | Positive |
| GX73 (type-A) | Positive |
| OGB (type-O) | Positive |
| Negative Control | |

**Table 2. Detection of Type O FMDV using the Type O-specific primer pair (SEQ ID No. 18 plus SEQ ID No. 21). The process is the same as that in the detection of all subtypes of FMDV except that specific primers are used.**

| **Sample ID** | **Result** |
|---|---|
| Reference Standard | |
| Assay negative | |
| SAT- 1 | Negative |
| SAT-2 | Negative |
| SAT-3 | Negative |
| GX73 (type-A) | Negative |
| OGB (type-O) | Positive |
| Negative Control | |

As shown in the above example, it can be realised that the detection kit may be used conveniently in various testing sites including farms. Furthermore, the detection kit is relatively easier to use than existing methods, and may be able to provide the detection results in a shorter time - the detection results may be available within one day if desired. As it is a RNA-based detection system, the specificity and the sensitivity may be enhanced - the detection kit is specific to FMDV, and the concentration of the FMDV in the sample may no longer be important, as the virus will be replicated to an amplified molecule for detection.

It will be apparent to one skilled in the art that the primers, detection probe, and capture probe may be also useful in the form of RNA molecules. DNA molecules are preferred due to stability reasons.

## Claims

1. A process for detecting foot-and-mouth disease virus (FMDV) in a biological sample, which comprises the following steps:
(A) isolating the RNA molecules of the FMDV from the biological sample;
(B) subjecting the RNA molecules of the FMDV to amplification based on a NASBA method to obtain an amplified product containing a nucleic acid sequence complementary to at least a portion of the RNA sequence of FMDV, wherein the NASBA-based amplification of the RNA of the FMDV comprises:
(a) annealing primer A to the isolated RNA molecules resulted in step (A), wherein primer A includes:
(i) a first DNA sequence for binding to at least a portion of the RNA sequence of FMDV,
(ii) a second DNA sequence encoding a promoter DNA sequence of an RNA polymerase;
(b) allowing the primer A to extend under the presence of a reverse transcriptase to obtain an RNA-DNA hybrid;
(c) digesting the RNA sequence in the RNA-DNA hybrid obtained in step (b) with an RNase to obtain a single strand DNA molecule;
(d) annealing the primer B to the single strand DNA obtained in step (c), wherein primer B includes:
(i') a first DNA sequence for binding to at least a portion of the RNA sequence of FMDV,
(ii') optionally, a second DNA sequence encoding a detectable DNA sequence ;
(e) allowing the primer B to extend under the presence of a reverse transcriptase to obtain a double strand DNA molecule;
(f) subjecting the double-strand DNA obtained in step (e) to amplification under the presence of an RNA polymerase to obtain an amplified RNA product
(C)detecting the amplified RNA product;
wherein the primer A comprises either one of the DNA sequence as set forth in SEQ ID Nos. 1, 2, 3, 4, 5 and 6, and wherein the primer B comprises either one of the DNA sequence as set forth in SEQ ID Nos. 8, 9, 10, 11, 12 or 13.

2. The process of claim 1, wherein the RNase is RNase H.

3. The process of claim 1, wherein the reverse transcriptase is an avian myeloblastosis virus reverse transcriptase.

4. The process of claim 1, wherein the RNA polymerase is a T7 RNA polymerase.

5. The process of claim 1, wherein the second DNA sequence of primer A encoding a promoter of an RNA polymerase comprises a sequence as set forth in SEQ ID No. 7.

6. The process of claim 1, wherein the detection of the amplified RNA products of step (C) includes: allowing a detection molecule to bind to the amplified RNA product, wherein the detection molecules comprises:
(i) a nucleic acid sequence encoding a portion of the FMDV RNA sequence that is complementary to that encoded in the amplification products; and
(ii) a signal generator selected from the group consisting of ruthenium- bipyridine complex, radioactive molecule, chemiluminescent molecule, fluorescent molecule and enzymatic molecule;
when primer B does not comprise the second DNA sequence encoding a DNA sequence for binding to the detection molecule; or
(i') a nucleic acid sequence complementary to a portion of the amplified RNA product that is complementary to the detectable sequence of primer B,
(ii') a signal generator selected from the group consisting of ruthenium- bipyridine complex, radioactive molecule, chemiluminescent molecule, fluorescent molecule and enzymatic molecule;
when primer B comprises the second DNA sequence encoding a DNA sequence for binding to the detection molecule

7. The process of claim 6, wherein the detection step of (C) further includes a step of binding the amplified RNA product to a capture probe before or when it is bound to the detection molecule, wherein the capture probe includes: a nucleic acid sequence complementary to a portion of sequence of the amplified RNA product and an immobiliser.

8. The process of claim 7, wherein the capture probe comprises a sequence as set forth in SEQ ID No. 24.

9. The process of claim 8, wherein the immobiliser is a magnetic particle attracted to a working electrode.

10. A kit for detecting foot-and-mouth disease virus (FMDV) in a biological samples, which comprises:
(A) an isolating agent for isolating the RNA molecules of FMDV from the biological sample;
(B) an amplifying agent for amplifying the isolated RNA molecules to obtain an amplified RNA product, wherein the amplified RNA product includes a nucleic acid sequence complementary to at least a portion of the RNA sequence of FMDV, wherein the amplifying agent includes:
(a) primer A which includes:
(i) a first DNA sequence for binding to at least a portion of the RNA sequence of FMDV, wherein the first DNA sequence of primer A comprises a DNA sequence as set forth in SEQ ID Nos. 1,2 , 3, 4, 5 or 6; and/or
(ii) a second DNA sequence encoding a promoter DNA sequence of a RNA polymerase;
(b) primer B which includes:
(i) a first DNA sequence encoding at least a portion of the RNA sequence of FMDV, wherein the first DNA sequence of primer B comprises a DNA sequence as set forth in SEQ ID Nos. 8, 9, 10, 11, 12 or 13; and
(ii) optionally, a second DNA sequence encoding a DNA sequence for binding to the detection molecule, wherein the second DNA sequence of primer B comprises a DNA sequence as set forth in SEQ ID No. 14; and/or
(c) a reverse transcriptase; and/or
(d) an RNase; and/or
(e) a RNA polymerase;
(C) a detecting agent for detecting the amplified RNA product.

11. The kit of claim 10, wherein the detecting agent comprises:
(a) a detection molecule which includes:
(i) a nucleic acid sequence encoding a portion of the FMDV RNA sequence that is complementary to that encoded in the amplification products; and
(ii) a signal generator selected from the group consisting of ruthenium- bipyridine complex, radioactive molecule, chemiluminescent molecule, fluorescent molecule and enzymatic molecule;
when primer B does not comprises the second DNA sequence encoding a DNA sequence for binding to the detection molecule; or
(i') a nucleic acid sequence complementary to the portion of the amplified RNA product that is complementary to the detectable sequence of primer B,
(ii') a signal generator selected from the group consisting of ruthenium- bipyridine complex, radioactive molecule, chemiluminescent molecule, fluorescent molecule and enzymatic molecule;
when primer B comprise the second DNA sequence encoding a DNA sequence for binding to the detection molecule.

12. The kit of claim 10, wherein the RNase is RNase H.

13. The kit of claim 10, wherein the reverse transcriptase is an avian myeloblastosis virus reverse transcriptase.

14. The kit of claim 10, wherein the RNA polymerase is an T7 RNA polymerase.

15. The kit of claim 10, which further includes a capture probe for capturing and immobilizing the amplified RNA product, wherein the capture probe includes: a nucleic acid sequence complementary to a portion of sequence of the amplified RNA product and an immobiliser.

16. The kit of claim 15, wherein the capture probe comprises a sequence as set forth in SEQ ID No. 24.

17. The kit of claim 15, wherein the immobiliser is a magnetic particle attracted to a working electrode.

18. The kit of claim 10, wherein the second DNA sequence of primer A comprises a DNA sequence as set forth in SEQ ID No. 7.

19. A DNA sequence, which includes:
(i) a first DNA sequence for binding to at least a portion of the RNA sequence of FMDV, wherein the first DNA sequence comprises a DNA sequence as set forth in SEQ ID Nos. 1, 2, 3, 4, 5 or 6; and
(ii) a second DNA sequence encoding a promoter DNA sequence of a RNA polymerase;
and the complementary sequence thereof.

20. The DNA sequence of claim 19, wherein the second sequence comprises a DNA sequence as set forth in SEQ ID No. 7.

21. A DNA sequence, which includes:
(i) a first DNA sequence encoding at least a portion of the RNA sequence of FMDV, wherein the first DNA sequence comprises a DNA sequence as set forth in SEQ ID Nos. 8, 9, 10, 11, 12 or 13; and
(ii) a second DNA sequence encoding a DNA sequence for binding to a detection molecule,
and the complementary sequence thereof.

22. The DNA sequence of claim 21, wherein the second DNA sequence comprises a DNA sequence set forth in SEQ ID No. 14.

## Patentansprüche

1. Verfahren zum Nachweis des Maul- und Klauenseuchevirus (MKSV) in einer biologischen Probe, bei dem man die folgenden Schritte durchführt:
(A) Isolieren der RNA-Moleküle des MKSV aus der biologischen Probe;
(B) Amplifizieren der RNA-Moleküle des MKSV auf der Grundlage einer NASBA-Methode, so dass ein amplifiziertes Produkt erhalten wird, das eine Nukleinsäuresequenz enthält, die zu wenigstens einem Teil der RNA-Sequenz des MKSV komplementär ist, wobei die Amplifikation der RNA des MKSV auf NASBA-Basis Folgendes umfasst:
(a) Annealing von Primer A an die in Schritt (A) erhaltenen isolierten RNA-Moleküle, wobei Primer A Folgendes enthält:
(i) eine erste DNA-Sequenz zur Bindung an wenigstens einen Teil der RNA-Sequenz des MKSV,
(ii) eine zweite DNA-Sequenz, die für eine Promotor-DNA-Sequenz einer RNA-Polymerase codiert;
(b) Verlängernlassen des Primers A in Gegenwart einer reversen Transkriptase, so dass ein RNA-DNA-Hybrid erhalten wird;
(c) Verdauen der RNA-Sequenz im in Schritt (b) erhaltenen RNA-DNA-Hybrid mit einer RNase, so dass ein einzelsträngiges DNA-Molekül erhalten wird;
(d) Annealing des Primers B an die in Schritt (c) erhaltene Einzelstrang-DNA, wobei Primer B Folgendes enthält:
(i') eine erste DNA-Sequenz zur Bindung an wenigstens einen Teil der RNA-Sequenz des MKSV,
(ii') gegebenenfalls eine zweite DNA-Sequenz, die für eine nachweisbare DNA-Sequenz codiert;
(e) Verlängernlassen des Primers B in Gegenwart einer reversen Transkriptase, so dass ein doppelsträngiges DNA-Molekül erhalten wird;
(f) Amplifizieren der in Schritt (e) erhaltenen Doppelstrang-DNA in Gegenwart einer RNA-Polymerase, so dass ein amplifiziertes RNA-Produkt erhalten wird;
(C) Nachweisen des amplifizierten RNA-Produkts;
wobei der Primer A eine der DNA-Sequenzen gemäß SEQ ID Nr. 1, 2, 3, 4, 5 und 6 und der Primer B eine der DNA-Sequenzen gemäß SEQ ID Nr. 8, 9, 10, 11, 12 oder 13 umfasst.

2. Verfahren nach Anspruch 1, wobei es sich bei der RNase um RNase H handelt.

3. Verfahren nach Anspruch 1, wobei es sich bei der reversen Transkriptase um eine reverse Transkriptase aus AMV (Avian Myeloblastosis Virus) handelt.

4. Verfahren nach Anspruch 1, wobei es sich bei der RNA-Polymerase um eine T7-RNA-Polymerase handelt.

5. Verfahren nach Anspruch 1, wobei die zweite DNA-Sequenz des Primers A, die für einen Promotor einer RNA-Polymerase codiert, eine Sequenz gemäß SEQ ID Nr. 7 umfasst.

6. Verfahren nach Anspruch 1, wobei der Nachweis der amplifizierten RNA-Produkte nach Schritt (C) Folgendes beinhaltet: Bindenlassen eines Nachweismoleküls an das amplifizierte RNA-Produkt, wobei das Nachweismolekül Folgendes umfasst:
(i) eine Nukleinsäuresequenz, die für einen Teil der MKSV-RNA-Sequenz codiert, der zur der in den Amplifikationsprodukten codierten Sequenz komplementär ist; und
(ii) einen Signalerzeuger, ausgewählt aus der Gruppe bestehend aus Ruthenium-Bipyridin-Komplex, radioaktivem Molekül, Chemilumineszenzmolekül, Fluoreszenzmolekül und enzymatischem Molekül;
wenn Primer B die zweite DNA-Sequenz, die für eine DNA-Sequenz zur Bindung an das Nachweismolekül codiert, nicht umfasst; oder
(i') eine Nukleinsäuresequenz, die zu einem Teil des amplifizierten RNA-Produkts komplementär ist, der zur der nachweisbaren Sequenz des Primers B komplementär ist,
(ii') einen Signalerzeuger, ausgewählt aus der Gruppe bestehend aus Ruthenium-Bipyridin-Komplex, radioaktivem Molekül, Chemilumineszenzmolekül, Fluoreszenzmolekül und enzymatischem Molekül;
wenn Primer B die zweite DNA-Sequenz, die für eine DNA-Sequenz zur Bindung an das Nachweismolekül codiert, umfasst.

7. Verfahren nach Anspruch 6, wobei der Nachweisschritt nach (C) ferner einen Schritt der Bindung des amplifizierten RNA-Produkts an eine Einfangsonde vor oder während seiner Bindung an das Nachweismolekül beinhaltet, wobei die Einfangsonde Folgendes enthält: eine zu einem Sequenzteil des amplifizierten RNA-Produkts komplementäre Nukleinsäuresequenz und einen Immobilisator.

8. Verfahren nach Anspruch 7, wobei die Einfangsonde eine Sequenz gemäß SEQ ID Nr. 24 umfasst.

9. Verfahren nach Anspruch 8, wobei es sich bei dem Immobilisator um ein magnetisches Teilchen handelt, das von einer Arbeitselektrode angezogen wird.

10. Kit zum Nachweis des Maul- und Klauenseuchevirus (MKSV) in einer biologischen Probe, der Folgendes umfasst:
(A) ein Isolierungsmittel zum Isolieren der RNA-Moleküle des MKSV aus der biologischen Probe;
(B) ein Amplifizierungsmittel zum Amplifizieren der isolierten RNA-Moleküle, so dass ein amplifiziertes RNA-Produkt erhalten wird, wobei das amplifizierte RNA-Produkt eine Nukleinsäuresequenz enthält, die zu wenigstens einem Teil der RNA-Sequenz des MKSV komplementär ist, wobei das Amplifizierungsmittel Folgendes enthält:
(a) Primer A, der Folgendes enthält:
(i) eine erste DNA-Sequenz zur Bindung an wenigstens einen Teil der RNA-Sequenz des MKSV, wobei die erste DNA-Sequenz des Primers A eine DNA-Sequenz gemäß SEQ ID Nr. 1, 2, 3, 4, 5 oder 6 umfasst; und/oder
(ii) eine zweite DNA-Sequenz, die für eine Promotor-DNA-Sequenz einer RNA-Polymerase codiert;
(b) Primer B, der Folgendes enthält:
(i) eine erste DNA-Sequenz, die für wenigstens einen Teil der RNA-Sequenz des MKSV codiert, wobei die erste DNA-Sequenz des Primers B eine DNA-Sequenz gemäß SEQ ID Nr. 8, 9, 10, 11, 12 oder 13 umfasst; und
(ii) gegebenenfalls eine zweite DNA-Sequenz, die für eine DNA-Sequenz zur Bindung an das Nachweismolekül codiert, wobei die zweite DNA-Sequenz des Primers B eine DNA-Sequenz gemäß SEQ ID Nr. 14 umfasst; und/oder
(c) eine reverse Transkriptase; und/oder
(d) eine RNase; und/oder
(e) eine RNA-Polymerase;
(C) ein Nachweismittel zum Nachweisen des amplifizierten RNA-Produkts.

11. Kit nach Anspruch 10, wobei das Nachweismittel Folgendes umfasst:
(a) ein Nachweismolekül, das Folgendes enthält:
(i) eine Nukleinsäuresequenz, die für einen Teil der MKSV-RNA-Sequenz codiert, der zur der in den Amplifikationsprodukten codierten Sequenz komplementär ist; und
(ii) einen Signalerzeuger, ausgewählt aus der Gruppe bestehend aus Ruthenium-Bipyridin-Komplex, radioaktivem Molekül, Chemilumineszenzmolekül, Fluoreszenzmolekül und enzymatischem Molekül;
wenn Primer B die zweite DNA-Sequenz, die für eine DNA-Sequenz zur Bindung an das Nachweismolekül codiert, nicht umfasst; oder
(i') eine Nukleinsäuresequenz, die zu einem Teil des amplifizierten RNA-Produkts komplementär ist, der zur der nachweisbaren Sequenz des Primers B komplementär ist,
(ii') einen Signalerzeuger, ausgewählt aus der Gruppe bestehend aus Ruthenium-Bipyridin-Komplex, radioaktivem Molekül, Chemilumineszenzmolekül, Fluoreszenzmolekül und enzymatischem Molekül;
wenn Primer B die zweite DNA-Sequenz, die für eine DNA-Sequenz zur Bindung an das Nachweismolekül codiert, umfasst.

12. Kit nach Anspruch 10, wobei es sich bei der RNase um RNase H handelt.

13. Kit nach Anspruch 10, wobei es sich bei der reversen Transkriptase um eine reverse Transkriptase aus AMV (Avian Myeloblastosis Virus) handelt.

14. Kit nach Anspruch 10, wobei es sich bei der RNA-Polymerase um eine T7-RNA-Polymerase handelt.

15. Kit nach Anspruch 10, der ferner eine Einfangsonde zum Einfangen und Immobilisieren des amplifizierten RNA-Produkts enthält, wobei die Einfangsonde Folgendes enthält: eine zu einem Sequenzteil des amplifizierten RNA-Produkts komplementäre Nukleinsäuresequenz und einen Immobilisator.

16. Kit nach Anspruch 15, wobei die Einfangsonde eine Sequenz gemäß SEQ ID Nr. 24 umfasst.

17. Kit nach Anspruch 15, wobei es sich bei dem Immobilisator um ein magnetisches Teilchen handelt, das von einer Arbeitselektrode angezogen wird.

18. Kit nach Anspruch 10, wobei die zweite DNA-Sequenz des Primers A eine DNA-Sequenz gemäß SEQ ID Nr. 7 umfasst.

19. DNA-Sequenz, die Folgendes enthält:
(i) eine erste DNA-Sequenz zur Bindung an wenigstens einen Teil der RNA-Sequenz des MKSV, wobei die erste DNA-Sequenz eine DNA-Sequenz gemäß SEQ ID Nr. 1, 2, 3, 4, 5 oder 6 umfasst; und
(ii) eine zweite DNA-Sequenz, die für eine Promotor-DNA-Sequenz einer RNA-Polymerase codiert; und die komplementäre Sequenz davon.

20. DNA-Sequenz nach Anspruch 19, wobei die zweite Sequenz eine DNA-Sequenz gemäß SEQ ID Nr. 7 umfasst.

21. DNA-Sequenz, die Folgendes enthält:
(i) eine erste DNA-Sequenz, die für wenigstens einen Teil der RNA-Sequenz des MKSV codiert, wobei die erste DNA-Sequenz eine DNA-Sequenz gemäß SEQ ID Nr. 8, 9, 10, 11, 12 oder 13 umfasst; und
(ii) eine zweite DNA-Sequenz, die für eine DNA-Sequenz zur Bindung an ein Nachweismolekül codiert,; und die komplementäre Sequenz davon.

22. DNA-Sequenz nach Anspruch 21, wobei die zweite DNA-Sequenz eine DNA-Sequenz gemäß SEQ ID Nr. 14 umfasst.

## Revendications

1. Processus pour détecter le virus de la fièvre aphteuse (FMDV) dans un échantillon biologique, lequel comprend les étapes suivantes :
(A) isoler les molécules d'ARN du FMDV de l'échantillon biologique ;
(B) soumettre les molécules d'ARN du FMDV à une amplification, basée sur un procédé NASBA, pour obtenir un produit amplifié contenant une séquence d'acide nucléique complémentaire d'une partie au moins de la séquence d'ARN du FMDV, dans lequel l'amplification, basée sur un NASBA, de l'ARN du FMDV comprend les étapes consistant à :
(a) anneler une amorce A avec les molécules d'ARN isolées résultant de l'étape (A), dans lequel l'amorce A comprend :
(i) une première séquence d'ADN destinée à se lier à une partie au moins de la séquence d'ARN du FMDV,
(ii) une deuxième séquence d'ADN codant pour une séquence d'ADN promoteur d'une ARN polymérase ;
(b) laisser l'amorce A s'allonger, en présence d'une transcriptase inverse, pour obtenir un hybride d'ARN-ADN ;
(c) digérer la séquence d'ARN dans l'hybride d'ARN-ADN obtenu à l'étape (b) avec une RNAse dans le but d'obtenir une molécule d'ADN en simple brin ;
(d) anneler l'amorce B avec l'ADN en simple brin obtenu à l'étape (c), dans lequel l'amorce B comprend :
(i') une première séquence d'ADN destinée à se lier à une partie au moins de la séquence d'ARN du FMDV,
(ii') facultativement, une deuxième séquence d'ADN codant pour une séquence d'ADN détectable ;
(e) laisser l'amorce B s'allonger, en présence d'une transcriptase inverse, pour obtenir une molécule d'ADN en double brin ;
(f) soumettre l'ADN en double brin obtenu à l'étape (e) à une amplification, en présence d'une ARN polymérase, dans le but d'obtenir un produit d'ARN amplifié ;
(C) détecter l'ARN amplifié ;
dans lequel l'amorce A comprend l'une ou l'autre des séquences d'ADN, telles qu'indiquées dans les SEQ ID n° 1, 2, 3, 4, 5 et 6, et dans lequel l'amorce B comprend l'une ou l'autre des séquences d'ADN telles qu'indiquées dans les SEQ ID n° 8, 9, 10, 11, 12 ou 13.

2. Processus selon la revendication 1, dans lequel la RNAse est une RNAse H.

3. Processus selon la revendication 1, dans lequel la transcriptase inverse est une transcriptase inverse de virus de myéloblaste aviaire.

4. Processus selon la revendication 1, dans lequel l'ARN polymérase est une ARN polymérase de T7.

5. Processus selon la revendication 1, dans lequel la deuxième séquence d'ADN de l'amorce A, codant pour un promoteur d'ARN polymérase, comprend une séquence telle qu'indiquée dans la SEQ ID n° 7.

6. Processus selon la revendication 1, dans lequel la détection des produits d'ARN amplifié de l'étape (C) comprend l'étape consistant à laisser une molécule de détection se lier au produit d'ARN amplifié, dans lequel les molécules de détection comprennent :
(i) une séquence d'acide nucléique, codant pour une partie de la séquence d'ARN du FMDV, qui est complémentaire de celle codée dans les produits d'amplification ; et
(ii) un générateur de signal choisi dans le groupe composé du complexe ruthénium-bipyridine, d'une molécule radioactive, d'une molécule chimiluminescente, d'une molécule fluorescente et d'une molécule enzymatique ; quand l'amorce B ne comprend pas la deuxième séquence d'ADN codant pour une séquence d'ADN destinée à se lier à la molécule de détection ; ou
(i') une séquence d'acide nucléique complémentaire d'une partie du produit d'ARN amplifié, qui est complémentaire de la séquence détectable de l'amorce B,
(ii') un générateur de signal choisi dans le groupe composé du complexe ruthénium-bipyridine, d'une molécule radioactive, d'une molécule chimiluminescente, d'une molécule fluorescente et d'une molécule enzymatique ; quand l'amorce B comprend la deuxième séquence d'ADN codant pour une séquence d'ADN destinée à se lier à la molécule de détection.

7. Processus selon la revendication 6, dans lequel l'étape de détection du paragraphe (C) comprend en outre une étape de liaison du produit d'ARN amplifié à une sonde de capture avant ou pendant qu'il se lie à la molécule de détection, dans lequel la sonde de capture comprend : une séquence d'acide nucléique complémentaire d'une partie de la séquence du produit d'ARN amplifié ainsi qu'un immobilisateur.

8. Processus selon la revendication 7, dans lequel la sonde de capture comprend une séquence telle qu'indiquée dans la SEQ ID n° 24.

9. Processus selon la revendication 8, dans lequel l'immobilisateur est une particule magnétique attirée vers une électrode en fonctionnement.

10. Kit pour détecter le virus de la fièvre aphteuse (FMDV) dans un échantillon biologique, lequel comprend :
(A) un agent d'isolement destiné à isoler les molécules d'ARN du FMDV provenant de l'échantillon biologique ;
(B) un agent d'amplification destiné à amplifier les molécules d'ARN isolées dans le but d'obtenir un produit d'ARN amplifié, dans lequel le produit d'ARN amplifié comprend une séquence d'acide nucléique complémentaire d'une partie au moins de la séquence d'ARN du FMDV, dans lequel l'agent d'amplification comprend :
(a) une amorce A qui comprend :
(i) une première séquence d'ADN destinée à se lier à une partie au moins de la séquence d'ARN du FMDV, dans lequel la première séquence d'ADN de l'amorce A comprend une séquence d'ADN telle qu'indiquée dans les SEQ ID n° 1, 2, 3, 4, 5 ou 6 ; et/ou
(ii) une deuxième séquence d'ADN codant pour une séquence d'ADN promoteur d'une ARN polymérase ;
(b) une amorce B qui comprend :
(i) une première séquence d'ADN codant pour une partie au moins de la séquence d'ARN du FMDV, dans lequel la première séquence d'ADN de l'amorce B comprend une séquence d'ADN telle qu'indiquée dans les SEQ ID n° 8, 9, 10, 11, 12 ou 13 ; et
(ii) facultativement, une deuxième séquence d'ADN codant pour une séquence d'ADN destinée à se lier à la molécule de détection, dans lequel la deuxième séquence d'ADN de l'amorce B comprend une séquence d'ADN telle qu'indiquée dans la SEQ ID n° 14 ; et/ou
(c) une transcriptase inverse ; et/ou
(d) une RNAse ; et/ou
(e) une ARN polymérase ;
(C) un agent de détection destiné à détecter le produit d'ARN amplifié.

11. Kit selon la revendication 10, dans lequel l'agent de détection comprend :
(a) une molécule de détection qui comprend :
(i) une séquence d'acide nucléique, codant pour une partie de la séquence d'ARN du FMDV, qui est complémentaire de celle codée dans les produits d'amplification ; et
(ii) un générateur de signal choisi dans le groupe composé du complexe ruthénium-bipyridine, d'une molécule radioactive, d'une molécule chimiluminescente, d'une molécule fluorescente et d'une molécule enzymatique ; quand l'amorce B ne comprend pas la deuxième séquence d'ADN codant pour une séquence d'ADN destinée à se lier à la molécule de détection ; ou
(i') une séquence d'acide nucléique complémentaire de la partie du produit d'ARN amplifié, qui est complémentaire de la séquence détectable de l'amorce B,
(ii') un générateur de signal choisi dans le groupe composé du complexe ruthénium-bipyridine, d'une molécule radioactive, d'une molécule chimiluminescente, d'une molécule fluorescente et d'une molécule enzymatique ; quand l'amorce B comprend la deuxième séquence d'ADN codant pour une séquence d'ADN destinée à se lier à la molécule de détection.

12. Kit selon la revendication 10, dans lequel la RNAse est une RNAse H.

13. Kit selon la revendication 10, dans lequel la transcriptase inverse est une transcriptase inverse de virus de myéloblaste aviaire.

14. Kit selon la revendication 10, dans lequel l'ARN polymérase est une ARN polymérase de T7.

15. Kit, selon la revendication 10, qui comprend en outre une sonde de capture destinée à capturer et à immobiliser le produit d'ARN amplifié, dans lequel la sonde de capture comprend : une séquence d'acide nucléique, complémentaire d'une partie de la séquence du produit d'ARN amplifié, ainsi qu'un immobilisateur.

16. Kit selon la revendication 15, dans lequel la sonde de capture comprend une séquence telle qu'indiquée dans la SEQ ID n° 24.

17. Kit selon la revendication 15, dans lequel l'immobilisateur est une particule magnétique attirée vers une électrode en fonctionnement.

18. Kit selon la revendication 10, dans lequel la deuxième séquence d'ADN de l'amorce A comprend une séquence d'ADN telle qu'indiquée dans la SEQ ID n° 7.

19. Séquence d'ADN qui comprend :
(i) une première séquence d'ADN destinée à se lier à une partie au moins de la séquence d'ARN du FMDV, dans lequel la première séquence d'ADN comprend une séquence d'ADN telle qu'indiquée dans les SEQ ID n° 1, 2, 3, 4, 5 ou 6 ; et
(ii) une deuxième séquence d'ADN codant pour une séquence d'ADN promoteur d'une ARN polymérase ; et la séquence complémentaire de celle-là.

20. Séquence d'ADN selon la revendication 19, dans laquelle la deuxième séquence comprend une séquence d'ADN telle qu'indiquée dans la SEQ ID n° 7.

21. Séquence d'ADN qui comprend :
(i) une première séquence d'ADN codant pour une partie au moins de la séquence d'ARN du FMDV, dans lequel la première séquence d'ADN comprend une séquence d'ADN telle qu'indiquée dans les SEQ ID n° 8, 9, 10, 11, 12 ou 13 ; et
(ii) une deuxième séquence d'ADN codant pour une séquence d'ADN destinée à se lier à une molécule de détection
et la séquence complémentaire de celle-là.

22. Séquence d'ADN selon la revendication 21, dans laquelle la deuxième séquence d'ADN comprend une séquence d'ADN telle qu'indiquée dans la SEQ ID n° 14.


### SEQUENCE LISTING

<110> HONG KONG DNA CHIPS LIMITED
<120> A PROCESS FOR DETECTING FOOT-AND-MOUTH DISEASE VIRUS AND A KIT THEREFOR
<130> P2001,0747
<140>
   <141>
<160> 25
<170> PatentIn Ver. 2.1
<210>1
   <211> 26
   <212> DNA
   <213> Artificial Sequence
<220>
   <223> Description of Artificial Sequence:probe
<400> 1
   agaaggcttc tcagatcccg agtgtc 26
<210> 2
   <211> 120
   <212> DNA
   <213> Artificial Sequence
<220>
   <223> Description of Artificial Sequence:primer
<400> 2
   tcaggcgtag aagcttttta aactgggcac tttaaagaag tcccaatccc cttctcagat 60
   cccgagtgtc ccgtgttacc tcggggtacc tgaagggcat ccttagcctg tcaccagtga 120
<210> 3
   <211> 160
   <212> DNA
   <213> Artificial Sequence
<220>
   <223> Description of Artificial Sequence:primer
<400> 3
   gccggcctcc ggtcgcctat tcaggcgtag aagcttttta aactgggcac tttaaagaag 60
   tcccaatccc cttctcagat cccgagtgtc ccgtgttacc tcggggtacc tgaagggcat 120
   ccttagcctg tcaccagtga ccgagtacca gtaccagtgt 160
<210> 4
   <211> 25
<212> DNA
   <213> Artificial Sequence
<220>
   <223> Description of Artificial Sequence:primer
<400> 4
   agaaggccag tccccttctc agatc 25
<210> 5
   <211> 119
   <212> DNA
   <213> Artificial Sequence
<220>
   <223> Description of Artificial Sequence:primer
<400> 5
   cacctattca ggcatagaag ctttttaaac cgagcgcttt tatagaagcc ccagtcccct 60
   tctcagatcc cgagtgtcgc gtgttacctc ggggtacctg aagggcatcc ttagcctgt 119
<210> 6
   <211> 159
   <212> DNA
   <213> Artificial Sequence
<220>
   <223> Description of Artificial Sequence:primer
<400> 6
   gaaaggtgcc gacctccggt cacctattca ggcatagaag ctttttaaac cgagcgcttt 60
   tatagaagcc ccagtcccct tctcagatcc cgagtgtcgc gtgttacctc ggggtacctg 120
   aagggcatcc ttagcctgtc accagtgtgt gggtaccag 159
<210> 7
   <211> 31
   <212> DNA
   <213> Artificial Sequence
<220>
   <223> Description of Artificial Sequence:primer
<400> 7
   aattctaata cgactcacta tagggagaag g 31
<210> 8
   <211> 19
   <212> DNA
   <213> Artificial Sequence
<220>
   <223> Description of Artificial Sequence:primer
<400> 8
   tgtttcgtag cggagcatg 19
<210> 9
   <211> 119
   <212> DNA
   <213> Artificial Sequence
<220>
   <223> Description of Artificial Sequence:primer
<400> 9
   gtgctcgact ccacgcccgg tccactggcg ggtgttagta gcagcactgt tgtttcgtag 60
   cggagcatgg tggccgtggg aactcctcct tggtgacaag ggcccacggg gccgaaagc 119
<210> 10
   <211> 159
   <212> DNA
   <213> Artificial Sequence
<220>
   <223> Description of Artificial Sequence:primer
<400> 10
   gaggggtgac actttgtact gtgctcgact ccacgcccgg tccactggcg ggtgttagta 60
   gcagcactgt tgtttcgtag cggagcatgg tggccgtggg aactcctcct tggtgacaag 120
   ggcccacggg gccgaaagcc acgtccaaac ggacccaac 159
<210> 11
   <211> 19
   <212> DNA
   <213> Artificial Sequence
<220>
   <223> Description of Artificial Sequence:primer
<400> 11
   gcctggtctt tccaggtct 19
<210> 12
   <211> 119
   <212> DNA
   <213> Artificial Sequence
<220>
   <223> Description of Artificial Sequence:primer
<400> 12
   gatttaagca ggtttccaca actgataaaa ctcgtgcaac ttgaaacccc gcctggtctt 60
   tccaggtcta gaggggtgac actttgtact gtgctcgact ccacgcccgg tccactggc 119
<210> 13
<211> 159
   <212> DNA
   <213> Artificial Sequence
<220>
   <223> Description of Artificial Sequence:primer
<400> 13
   aggaacactt gtacaaacac gatttaagca ggtttccaca actgataaaa ctcgtgcaac 60
   ttgaaacccc gcctggtctt tccaggtcta gaggggtgac actttgtact gtgctcgact 120
   ccacgcccgg tccactggcg ggtgttagta gcagcactg 159
<210> 14
   <211> 20
   <212> DNA
   <213> Artificial Sequence
<220>
   <223> Description of Artificial Sequence:primer
<400> 14
   gatgcaaggt cgcatatgag 20
<210> 15
   <211> 30
   <212> DNA
   <213> Artificial Sequence
<220>
   <223> Description of Artificial Sequence:primer
<400> 15
   agaagggctg ttgggttggt ggtgttgtcc 30
<210> 16
   <211> 124
   <212> DNA
   <213> Artificial Sequence
<220>
   <223> Description of Artificial Sequence:primer
<400> 16
   gtggtgccgt gtatggtaga gcaagtcggg taagcggtgc cttgtggtat gctgttgggt 60
   tggtggtgtt gtccaaagct gtttctggcg ctccgttcgg gacccacgtg aggtccccct 120
   cgtg 124
<210> 17
   <211> 164
   <212> DNA
   <213> Artificial Sequence
<220>
   <223> Description of Artificial Sequence:primer
   <400> 17
   aacagttgcc aacacacggt gtggtgccgt gtatggtaga gcaagtcggg taagcggtgc 60
   cttgtggtat gctgttgggt tggtggtgtt gtccaaagct gtttctggcg ctccgttcgg 120
   gacccacgtg aggtccccct cgtgtttcac cgccacttct aagt 164
<210> 18
   <211> 30
   <212> DNA
   <213> Artificial Sequence
<220>
   <223> Description of Artificial Sequence:primer
<400> 18
   agaaggtctt tctgccttct gagccaacac 30
   <210> 19
   <211> 124
   <212> DNA
   <213> Artificial Sequence
<220>
   <223> Description of Artificial Sequence:primer
   <400> 19
   gtcacccggg ttgccttaat ggcaccatag ttaaaggagg tgggcagagc tctttctgcc 60
   ttctgagcca acacttgaag gtcacccctc acgttgctca cgcgggcgtc actgtactta 120
   caac 124
<210> 20
   <211> 164
   <212> DNA
   <213> Artificial Sequence
<220>
   <223> Description of Artificial Sequence:primer
   <400> 20
   tcattcggta gagtaactca gtcacccggg ttgccttaat ggcaccatag ttaaaggagg 60
   tgggcagagc tctttctgcc ttctgagcca acacttgaag gtcacccctc acgttgctca 120
   cgcgggcgtc actgtactta caacttccgt tgtagacggt cgct 164
<210> 21
   <211> 20
   <212> DNA
   <213> Artificial Sequence
<220>
   <223> Description of Artificial Sequence:primer
<400> 21
   gagaactacg gtggtgagac 20
<210> 22
   <211> 120
   <212> DNA
   <213> Artificial Sequence
<220>
   <223> Description of Artificial Sequence:primer
<400> 22
   cccaaaccac ctctgcgggt gagtctgcgg accccgtgac tgccaccgtc gagaactacg 60
   gtggtgagac acaagtccag aggcgccaag acacggacat tgcgttcata ttggacaggt 120
<210> 23
   <211>160
   <212> DNA
   <213> Artificial Sequence
<220>
   <223> Description of Artificial Sequence:primer
<400> 23
   tctgccggtc gacgccgaac cccaaaccac ctctgcgggt gagtctgcgg accccgtgac 60
   tgccaccgtc gagaactacg gtggtgagac acaagtccag aggcgccaag acacggacat 120
   tgcgttcata ttggacaggt tcgtgaaagt caagccaaag 160
<210> 24
   <211> 25
   <212> DNA
   <213> Artificial Sequence
<220>
   <223> Description of Artificial Sequence:probe
<400> 24
   gatgcccttc aggtaccccg aggta 25
<210> 25
   <211> 21
   <212> DNA
   <213> Artificial Sequence
<220>
   <223> Description of Artificial Sequence:probe
<400> 25
   tggacctgat gcagatccct g 21